# FASCICULE DE BREVET EUROPEEN

(11) **EP 1 752 095 B1**
(45) Date de publication et mention de la délivrance du brevet: **25.02.2009**
(21) Numéro de dépôt: 06118553.4
(22) Date de dépôt: 07.08.2006
(51) Int. Cl.: A61B 5/15

(54) **Dispositif de prélèvement de fluide corporel ou de perfusion a moyen de protection**
Gerät zur Entnahme von Körperflüssigkeiten mit Schutzvorrichtung
Device for taking body fluids having protecting means

(30) Priorité: 08.08.2005 FR 0508501
(43) Date de publication de la demande: 14.02.2007
(73) Titulaire: P2A Medical, 73410 Albens (FR)
(72) Inventeur: Orlu, Alain, 74540 Viuz la Chiesaz (FR); Commaret, Pierre-André, 1279 Bogis Bossey (CH)
(74) Mandataire: Poncet, Jean-François

(56) Documents cités:
- EP-A- 0 363 180
- EP-A- 0 369 619
- EP-A- 0 539 634
- WO-A-89/04141

## Description

La présente invention concerne les dispositifs de prélèvement de fluide corporel ou de perfusion utilisés dans le milieu médical, et plus particulièrement les dispositifs de prélèvement de fluide corporel ou de perfusion comportant des moyens de protection.

Les dispositifs de prélèvement de fluide corporel ou de perfusion couramment utilisés dans le milieu médical comportent généralement une aiguille destinée à pénétrer dans le corps d'un patient, par exemple dans une veine. Une fois le prélèvement de fluide corporel ou la perfusion réalisée, l'aiguille est retirée du corps du patient et se retrouve généralement porteuse de sang ou autre fluide corporel du patient. Dans l'hypothèse où le patient serait atteint d'une infection transmissible, ce sang ou autre fluide corporel serait une source de contamination. Or les infections dont le patient peut être porteur ne sont pas toujours décelées ou décelables.

Il est dès lors nécessaire d'assurer systématiquement une protection efficace du personnel médical contre des blessures pouvant être provoquées par cette aiguille contaminée avec du sang ou autre fluide corporel. Il est aussi nécessaire d'éviter que le sang ou autre fluide corporel contamine l'environnement, tel que le mobilier ou les autres instruments médicaux par exemple.

Pour ce faire, on connaît déjà des capuchons tubulaires borgnes que l'on adapte pour entourer l'aiguille du dispositif de prélèvement de fluide corporel ou de perfusion après la perfusion ou le prélèvement.

Ces capuchons tubulaires borgnes comportent un logement intérieur destiné à contenir la partie dépassante de l'aiguille. Le diamètre du logement intérieur est très peu supérieur au diamètre de l'aiguille. Ces capuchons tubulaires borgnes présentent en outre généralement une épaisseur de paroi latérale très faible.

Ce faible diamètre intérieur et cette faible épaisseur de paroi latérale obligent le personnel médical à utiliser deux mains pour venir loger l'aiguille dans son capuchon tubulaire borgne de protection, une première main tenant le capuchon, une seconde main tenant l'aiguille. L'insertion de l'aiguille dans un logement de diamètre très peu supérieur à son propre diamètre requiert une grande dextérité, mais encore une grande prudence, car si la personne venait à manquer le logement interne du capuchon tubulaire borgne avec l'aiguille, il y a de fortes chances que celle-ci vienne se ficher dans un doigt de la première main tenant le capuchon, du fait de la faible épaisseur de la paroi latérale du capuchon tubulaire borgne.

L'utilisation des capuchons tubulaires borgnes généralement connus s'avère donc délicate pour le personnel médical et non dépourvue de tout risque d'accident pouvant entraîner des maladies parfois graves par contamination avec le sang ou autre fluide corporel porté par l'aiguille.

Malgré le risque, et du fait de ces difficultés d'utilisation des capuchons tubulaires borgnes pour la protection des aiguilles des dispositifs de prélèvement de fluide corporel ou de perfusion, le personnel médical omet la plupart du temps volontairement de les utiliser. En pratique, il garde alors en main le dispositif de prélèvement ou de perfusion muni de son aiguille (non protégée et contaminée avec du sang) à l'air libre, ou pose le dispositif de prélèvement ou de perfusion muni de son aiguille souillée par du sang du patient sur une table ou dans un récipient contenant d'autres instruments médicaux. Il y a donc un fort risque que le personnel médical se blesse avec l'aiguille souillée avec du sang du patient ou blesse une personne présente dans la salle où il exerce son activité. Il y a également un fort risque de contaminer les instruments médicaux et de façon générale l'environnement de travail.

En outre, le personnel médical a un fort risque de se tacher avec le sang présent sur l'aiguille qui vient d'être retirée du corps du patient.

Le document WO 2004/000397 décrit un dispositif de prélèvement de fluide corporel ou de perfusion à système de protection automatique. Dans ce document, un tube recouvre la partie dépassante d'une aiguille et coulisse pour la découvrir au fur et à mesure de la pénétration de l'aiguille dans le corps du patient. Le tube vient ensuite recouvrir à nouveau la partie dépassante de l'aiguille au fur et à mesure que celle-ci est retirée du corps du patient. Un tel système est peu maniable, imprécis lors de la piqûre du patient, et peut donc provoquer des maladresses de manipulation par le personnel hospitalier, provoquant la souffrance inutile du patient. Ce système comporte en outre de nombreuses pièces, dont un ressort, et son assemblage est complexe et long.

Le document EP 0 363 180 décrit un dispositif de prélèvement de fluide corporel ou de perfusion à moyen de protection, comportant :
- un corps central longitudinal creux, permettant le passage d'un fluide entre une première et une seconde extrémité, muni à son extrémité proximale de moyens de connexion à une ligne de perfusion ou de prélèvement, et solidaire d'une aiguille coaxiale qui dépasse de son extrémité distale,
- un corps externe longitudinal creux, solidaire du corps central et disposé de façon concentrique autour du corps central, et conformé pour constituer un moyen de préhension pour un utilisateur,
- un passage annulaire prévu entre le corps central et le corps externe,
- un tube de protection à fente longitudinale interrompue, monté à coulissement longitudinal dans le passage annulaire entre le corps externe et le corps central, muni d'une extrémité proximale de préhension pour être déplacé sélectivement entre d'une part une position proximale de retrait dans laquelle l'aiguille n'est pas ou très peu recouverte par le tube de protection, et d'autre part une position distale de protection dans laquelle le tube de protection recouvre l'aiguille sur toute sa longueur.

Dans ce document, le corps central est nécessairement une pièce distincte du corps externe et doit être rendu solidaire du corps externe par un axe rapporté de faibles dimensions qui est inséré en force ou collé dans des trous transversaux du corps central et du corps externe et qui traverse librement une fente longitudinale interrompue du tube de protection. Du fait du grand nombre de pièces à fabriquer puis à assembler, et du fait qu'une telle opération d'assemblage est difficile et longue, cela rend le prix de vente trop élevé pour un dispositif à usage unique.

Le document EP 0 369 619 décrit un dispositif inspiré du dispositif du document EP 0 363 180 et qui comporte les mêmes inconvénients de montage.

Un premier problème proposé par l'invention est de concevoir un système de protection fiable, peu onéreux, facile à manier d'une seule main par le personnel médical.

L'invention cherche plus particulièrement à concevoir un tel dispositif de prélèvement ou de perfusion dont le montage et la fabrication sont simples et peu onéreux.

Selon un autre aspect, l'invention vise à concevoir un dispositif de prélèvement ou de perfusion à moyen de protection dont l'utilisation ne présente aucun risque de contamination ni pour le personnel médical ni pour l'environnement de travail, en évitant toute manipulation du moyen de protection par la main de l'utilisateur au voisinage de l'extrémité dangereuse de l'aiguille et en évitant tout mouvement allant à l'encontre de l'extrémité dangereuse de l'aiguille.

Simultanément, l'invention vise à éviter tout risque de perte par l'utilisateur du moyen de protection.

Simultanément, l'invention cherche à concevoir un dispositif qui n'oblige pas le personnel médical à effectuer des gestes compliqués ou non usuels dans son métier pour son utilisation.

L'invention cherche en outre à concevoir un dispositif de prélèvement ou de perfusion à moyen de protection pratique, hygiénique et n'autorisant qu'un usage unique du dispositif de prélèvement ou de perfusion.

Pour atteindre ces objets, ainsi que d'autres, l'invention propose un dispositif de prélèvement de fluide corporel ou de perfusion à moyen de protection, comportant :
- un corps central longitudinal creux, permettant le passage d'un fluide entre une première et une seconde extrémité, muni à son extrémité proximale de moyens de connexion à une ligne de perfusion ou de prélèvement, et solidaire d'une aiguille coaxiale qui dépasse de son extrémité distale,
- des moyens pour recouvrir sélectivement la partie dépassante d'aiguille après usage,
- un corps externe longitudinal creux à paroi périphérique tubulaire, solidaire du corps central et disposé de façon concentrique autour du corps central, et conformé pour constituer un moyen de préhension pour un utilisateur,
- un passage annulaire prévu entre le corps central et le corps externe,
- un tube de protection ouvert à son extrémité distale et monté à coulissement longitudinal dans le passage annulaire entre le corps externe et le corps central, muni d'une extrémité proximale de préhension pour être déplacé sélectivement entre d'une part une position proximale de retrait dans laquelle l'aiguille n'est pas ou très peu recouverte par le tube de protection de telle sorte que celle-ci est utilisable pour un prélèvement ou une perfusion, et d'autre part une position distale de protection dans laquelle le tube de protection recouvre l'aiguille sur toute sa longueur,
- au moins un pont radial de fixation, rendant le corps externe solidaire du corps central en se développant radialement depuis le corps central jusqu'au corps externe,
- au moins une fente longitudinale, sur une partie au moins de la longueur du tube de protection, dans laquelle coulisse ledit au moins un pont radial de fixation lors du mouvement de coulissement du tube de protection par rapport aux corps central et externe,
   dans lequel :

- le corps central, le pont radial de fixation et le corps externe sont d'une seule pièce,
- la fente longitudinale se développe jusqu'à l'extrémité distale du tube de protection.

Un tel système assure une protection efficace de l'aiguille et des utilisateurs. En outre, son usage est simple et rapide, et ne nécessite l'usage que d'une seule main de l'utilisateur. On évite en effet toute manipulation du moyen de protection par la main de l'utilisateur au voisinage de l'extrémité dangereuse de l'aiguille, et surtout tout mouvement allant à l'encontre de l'extrémité dangereuse de l'aiguille pouvant amener l'utilisateur à se planter l'aiguille dans une main.

Il est également impossible que l'utilisateur perde le moyen de protection, puisque celui-ci est solidaire du dispositif de prélèvement ou de perfusion. Le moyen de protection est ainsi toujours disponible.

La réalisation en une seule pièce du corps central, du pont radial de fixation et du corps externe s'avère particulièrement judicieuse d'un point de vue économique. Il n'y a en effet alors plus à assembler de multiples pièces de faible encombrement et susceptibles d'être perdues, ce qui contribue à réduire considérablement les coûts d'assemblage et donc de fabrication du dispositif.

Le fait que la fente longitudinale du tube de protection se développe jusqu'à l'extrémité distale de celui-ci permet de monter le tube de protection dans le passage annulaire prévu entre le corps central et le corps externe par une simple translation, car le pont radial de fixation peut s'engager dans la fente longitudinale du tube de protection par son extrémité distale. Le montage du tube de protection est donc très simple, rapide et sans difficulté.

Avantageusement, l'extrémité proximale de préhension du tube de protection peut comporter au moins une patte radiale de poussée s'étendant radialement à l'écart du tube de protection.

Le dispositif de prélèvement ou de perfusion selon l'invention comporte ainsi des moyens de préhension semblables à ceux d'une seringue classique. Ceci garantit que le personnel médical n'ait à utiliser qu'une seule main pour effectuer la mise en oeuvre du moyen de protection selon l'invention, par un geste facile et très usité dans le cadre des professions médicales, donc sans risque de mauvaise manipulation.

Les moyens de préhension ainsi constitués sont en outre faciles à réaliser et peu onéreux.

De manière avantageuse, le dispositif de prélèvement ou de perfusion à moyen de protection selon l'invention peut comporter des moyens de verrouillage du tube de protection en position distale de protection et/ou en position proximale de retrait.

Le verrouillage du tube de protection en position proximale de retrait permet l'utilisation du dispositif de prélèvement ou de perfusion sans risque de conflit entre le moyen de protection comportant le tube de protection et le milieu environnant. Par exemple, le tube de protection ne coulissera ainsi pas de manière intempestive au voisinage du corps du patient lors d'un prélèvement ou d'une perfusion. On garantit ainsi la qualité de la piqûre réalisée par le personnel médical.

Le verrouillage en position distale de protection du tube de protection permet d'éviter efficacement tout risque de coulissement intempestif du tube de protection pouvant mener à redécouvrir l'aiguille du dispositif de prélèvement ou de perfusion une fois que celle-ci a été utilisée puis recouverte sur la totalité de sa longueur par le tube de protection.

De préférence, les moyens de verrouillage dans la position proximale de retrait peuvent comporter :
- une première encoche annulaire périphérique pratiquée sur le tube de protection au voisinage d'une extrémité distale du tube de protection,
- au moins une patte de verrouillage élastique radiale s'étendant radialement depuis une extrémité distale du corps externe, et venant s'engager dans la première encoche annulaire périphérique lorsque le tube de protection est en position proximale de retrait.

De même, les moyens de verrouillage dans la position distale de protection peuvent avantageusement comporter :
- une seconde encoche annulaire périphérique ou un épaulement à face tournée vers l'extrémité proximale de préhension, pratiquée sur le tube de protection au voisinage de l'extrémité proximale de préhension du tube de protection,
- au moins une patte de verrouillage élastique radiale s'étendant radialement depuis l'extrémité distale du corps externe, et venant se loger dans la seconde encoche annulaire périphérique ou sur l'épaulement lorsque le tube de protection est en position distale de protection.

On réalise ainsi des moyens de verrouillage dans la position proximale de retrait et des moyens de verrouillage dans la position distale de protection de façon simple, pratique, économique et surtout compacte, toujours afin de ne pas encombrer inutilement l'espace de travail du personnel médical, tant pour des raisons de qualité de travail que pour des raisons de sécurité pour le personnel médical. On facilite également la tenue en main et le maniement du dispositif de prélèvement ou de perfusion selon l'invention.

Selon un autre mode de réalisation, les moyens de verrouillage dans la position proximale de retrait peuvent comporter :
- au moins un premier plot d'arrêt s'étendant radialement à l'écart du tube de protection au voisinage de l'extrémité distale du tube de protection,
- au moins une patte de verrouillage longitudinale dans une lumière de la paroi périphérique tubulaire du corps externe, apte à se défléchir élastiquement radialement, à extrémité libre orientée vers l'extrémité proximale du corps externe apte à venir en butée contre le premier plot d'arrêt lorsque le tube de protection est en position proximale de retrait.

Dans cet autre mode de réalisation, les moyens de verrouillage dans la position distale de protection peuvent avantageusement comporter :
- au moins un second plot d'arrêt s'étendant radialement à l'écart du tube de protection au voisinage de l'extrémité proximale du tube de protection,
- une lumière dans la patte de verrouillage, apte à recevoir le second plot d'arrêt lorsque le tube de protection est en position distale de protection.

De tels moyens de verrouillage sont faciles à fabriquer, facilement démoulables, fiables et efficaces dans leur verrouillage du tube de protection.

Selon l'invention, le dispositif de prélèvement ou de perfusion à moyen de protection peut comporter un capuchon de protection tubulaire borgne amovible conformé pour s'emmancher sur l'extrémité distale du corps externe en entourant la partie dépassante d'aiguille, et de diamètre suffisant pour recevoir le tronçon distal dépassant du tube de protection en position distale de protection.

D'une part le capuchon peut protéger l'aiguille avant usage.

D'autre part la présence de ce capuchon de protection tubulaire borgne amovible, emmanché sur l'extrémité distale du corps externe qui entoure la partie dépassante de l'aiguille après usage, permet d'assurer une étanchéité parfaite et est apte à recueillir toute goutte de sang pouvant tomber de l'aiguille contaminée avec du sang du patient.

De préférence, le capuchon de protection tubulaire borgne, emmanché sur l'extrémité distale du corps externe, peut s'opposer à l'expansion radiale élastique de ladite au moins une patte de verrouillage. On interdit ainsi le déverrouillage et le coulissement du tube de protection avant usage.

Selon l'invention, le capuchon de protection tubulaire borgne peut comporter dans son fond des moyens de retenue du tube de protection en position distale de protection.

On évite ainsi tout risque de désemmancher le capuchon de protection tubulaire borgne une fois celui-ci emmanché sur l'extrémité distale du corps externe après usage.

Avantageusement, les moyens de retenue du tube de protection peuvent être conformés pour venir s'engager dans une première encoche annulaire périphérique du tube de protection lorsque le tube de protection est en position distale de protection et que le capuchon de protection est emmanché sur l'extrémité distale du corps externe.

On réalise ainsi de façon pratique et économique les moyens de retenue du tube de protection, après usage, garantissant l'usage unique du dispositif.

Avantageusement, le corps central, le corps externe et le capuchon de protection peuvent être en matière plastique, ce qui permet de disposer d'un dispositif de poids réduit et à faible coût de production.

D'autres objets, caractéristiques et avantages de la présente invention ressortiront de la description suivante de modes de réalisation particuliers, faite en relation avec les figures jointes, parmi lesquelles :
- la figure 1 est une vue en coupe longitudinale d'un premier mode de réalisation d'un dispositif de prélèvement ou de perfusion à moyen de protection, avec un tube de protection en position proximale de retrait ;
- la figure 2 est une vue de côté du dispositif de prélèvement ou de perfusion à moyen de protection de la figure 1 selon un plan de vue décalé de 90° par rapport au plan de vue de la figure 1 ;
- la figure 3 est une vue en coupe longitudinale du dispositif de prélèvement ou de perfusion à moyen de protection des figures 1 et 2, avec un tube de protection en position distale de protection ;
- la figure 4 est une vue en coupe longitudinale du dispositif de prélèvement ou de perfusion à moyen de protection des figures 1 à 3, avec un tube de protection en position distale de protection et muni d'un capuchon de protection tubulaire borgne ;
- la figure 5 est une vue en coupe transversale du dispositif de prélèvement ou de perfusion à moyen de protection des figures 1 à 4 ;
- la figure 6 est une vue de face d'un second mode de réalisation d'un dispositif de prélèvement ou de perfusion à moyen de protection ;
- la figure 7 est une vue de face en coupe longitudinale du dispositif de la figure 6;
- la figure 8 est une vue de côté du dispositif de la figure 6 selon un plan de vue décalé de 90° par rapport au plan de vue de la figure 6, avec un tube de protection en position proximale de retrait ;
- la figure 9 est une vue en perspective du dispositif des figures 6 à 8 ;
- la figure 10 est une vue du dispositif en coupe transversale selon le plan A-A de la figure 6, sans le capuchon de protection tubulaire borgne ; et
- la figure 11 est une vue en coupe longitudinale du dispositif des figures 6 à 10, avec un tube de protection en position distale de protection.

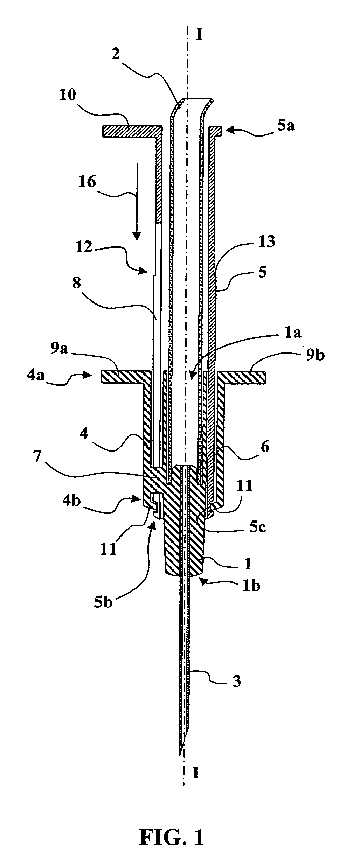

Dans les modes de réalisation illustrés sur les figures 1 à 11, un dispositif de prélèvement ou de perfusion à moyen de protection selon l'invention comporte un corps central 1 longitudinal creux, permettant le passage d'un fluide corporel comme du sang entre une première et une seconde extrémité, muni à son extrémité proximale 1 a de moyens de connexion à une ligne de perfusion ou de prélèvement 2, et solidaire d'une aiguille 3 coaxiale qui dépasse de son extrémité distale 1b. Un corps externe 4 longitudinal creux, solidaire du corps central 1, est disposé de façon concentrique autour du corps central 1, et est conformé pour constituer un moyen de préhension pour un utilisateur. Un tube de protection 5 permet de recouvrir sélectivement la partie dépassante d'aiguille 3 après usage, en étant monté à coulissement longitudinal selon l'axe |-| dans un passage annulaire 6 prévu entre le corps central 1 et le corps externe 4.

Le corps central 1 est surmoulé sur l'aiguille 3.

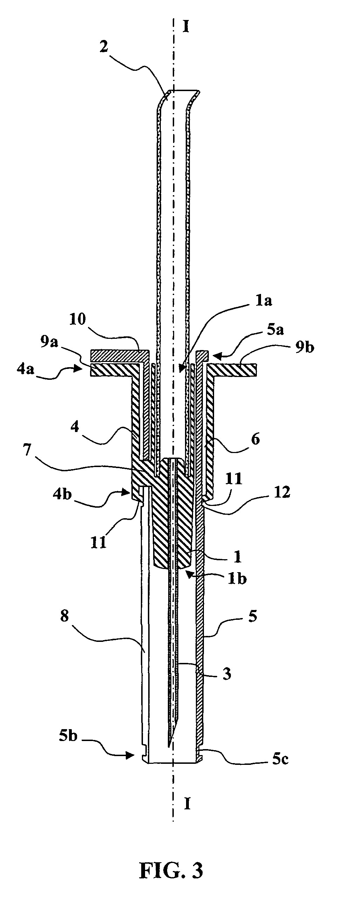

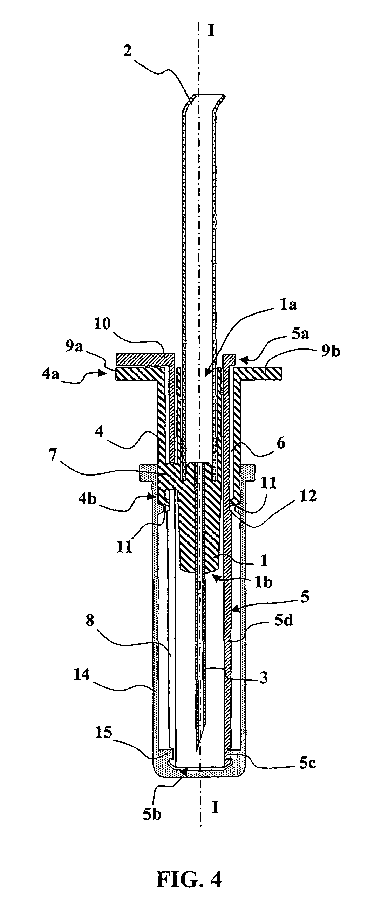

Le tube de protection 5 est muni d'une extrémité proximale de préhension 5a pour être déplacé sélectivement entre une position proximale de retrait (figure 1) dans laquelle l'aiguille 3 n'est pas ou très peu recouverte par le tube de protection 5 de telle sorte que celle-ci est utilisable pour un prélèvement ou une perfusion, et une position distale de protection (figures 3 et 4) dans laquelle le tube de protection 5 recouvre l'aiguille 3 sur toute sa longueur.

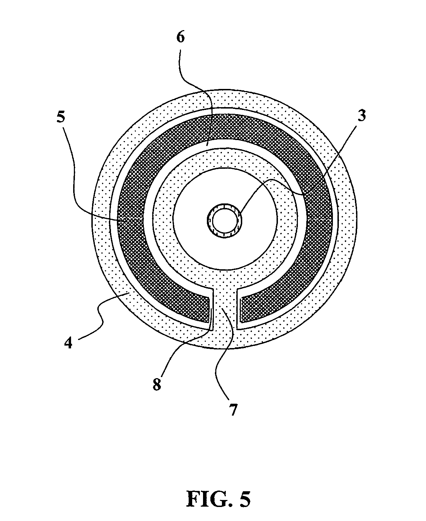

On distingue plus particulièrement au moyen des figures 1 et 5 que le corps externe 4 est rendu solidaire du corps central 1 par un pont radial 7 de fixation, se développant radialement depuis le corps central 1 jusqu'au corps externe 4. Le tube de protection 5 peut alors être monté à coulissement longitudinal dans le passage annulaire 6 laissé entre le corps central 1 et le corps externe 4.

Le corps central 1, le pont radial 7 de fixation et le corps externe 4 sont d'une seule pièce. Le fait de fabriquer de façon monobloc le corps central 1, le pont radial 7 de liaison et le corps externe 4 permet de diminuer considérablement le temps d'assemblage et de fabrication du dispositif de prélèvement ou de perfusion, et donc d'en baisser le coût.

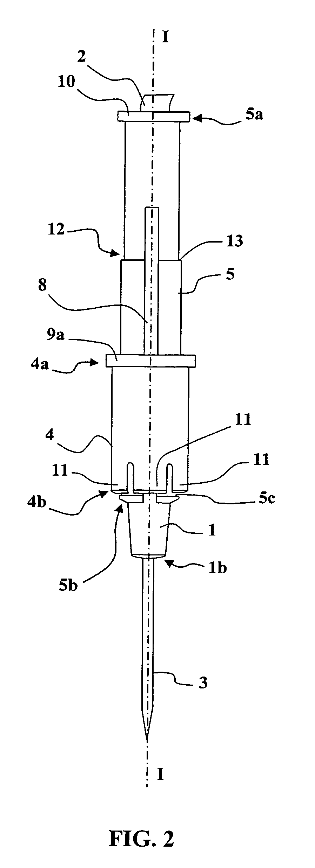

Pour que le tube de protection 5 puisse coulisser longitudinalement selon l'axe |-|, celui-ci comporte, sur une partie au moins de sa longueur, au moins une fente longitudinale 8 dans laquelle coulisse ledit pont radial 7 de fixation lors du mouvement de coulissement longitudinal du tube de protection 5 par rapport aux corps central 1 et externe 4. Cette fente longitudinale 8 est représentée de façon plus visible sur la figure 2.

La fente longitudinale 8 se développe jusqu'à l'extrémité distale 5b du tube de protection 5. Ceci permet de monter le tube de protection 5 dans le passage annulaire 6 prévu entre le corps central 1 et le corps externe 4 de façon simple par une simple translation d'axe I-I.

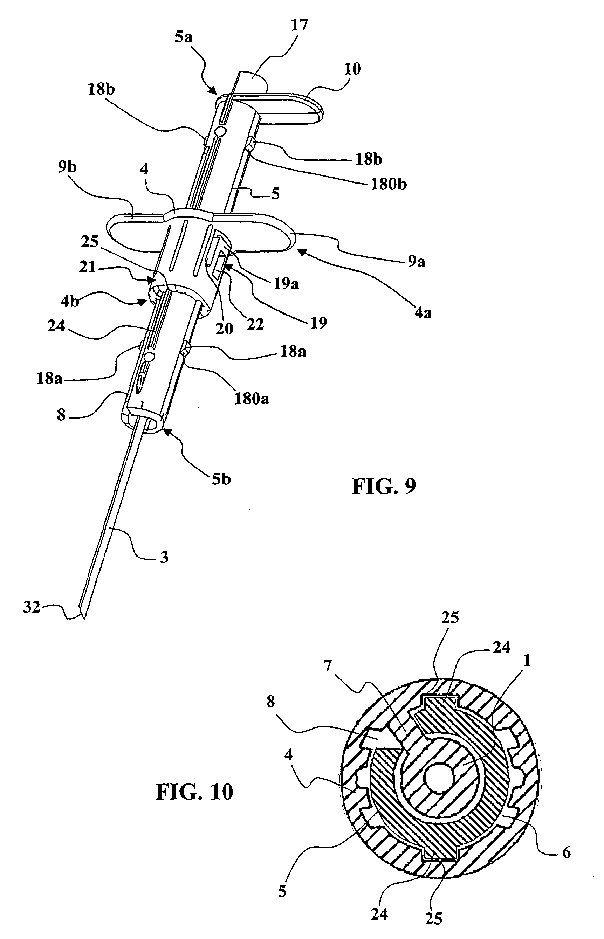

Dans les modes de réalisation illustrés sur les figures 1 à 11, le corps externe 4 est rendu solidaire du corps central 1 par seulement un pont radial 7 de fixation (figures 5 et 10). Il va de soi qu'il est possible de réaliser plus d'un pont radial 7 pour solidariser le corps central 1 et le corps externe 4.

Le tube de protection 5 étant fendu sur une grande partie de sa longueur, et la fente longitudinale 8 s'étendant jusqu'à l'extrémité distale 5b du tube de protection 5, il se peut que le tube de protection 5 manque de rigidité. Ce manque de rigidité pourrait nuire au bon coulissement axial du tube de protection 5 dans le passage annulaire 6 prévu entre le corps central 1 et le corps externe 4 (figures 5 et 10). Afin d'améliorer la rigidité du tube de protection 5 et d'améliorer son guidage lors de son coulissement d'axe |-|, il est prévu dans le second mode de réalisation de l'invention que le tube de protection 5 comporte des nervures longitudinales 24 (figures 9 et 10), aptes à coopérer avec des rainures longitudinales 25 du corps externe 4.

Dans le dispositif de prélèvement ou de perfusion à moyen de protection selon l'invention tel qu'illustré sur les figures 1 à 11, le corps externe 4 comporte deux pattes de préhension radiales 9a et 9b s'étendant radialement à l'écart du corps externe 4 et prévues au voisinage de l'extrémité proximale 4a du corps externe 4. L'extrémité proximale de préhension 5a du tube de protection 5 comporte une patte radiale de poussée 10 s'étendant radialement à l'écart du tube de protection 5.

On réalise ainsi des moyens de préhension du dispositif de prélèvement ou de perfusion à moyen de protection qui sont semblables à ceux d'une seringue classique, de telle sorte que le personnel médical utilisant ce dispositif n'a aucune difficulté à s'en saisir ni à l'utiliser. En effet, l'utilisation d'une seringue dans le milieu médical est courante, et la préhension du dispositif de prélèvement ou de perfusion à moyen de protection selon l'invention utilise avantageusement un geste facile est très usité par le personnel médical. On évite ainsi tout risque de manipulation lié à un mode de préhension compliqué ou inusité dans le cadre des professions médicales.

En outre, l'utilisateur peut tenir le dispositif de prélèvement ou de perfusion à moyen de protection selon l'invention avec une seule main, et mettre en mouvement le tube de protection 5 par rapport au corps externe 4 et au corps central 1 toujours avec cette unique main restant nettement à l'écart de l'aiguille 3. On libère ainsi une main de l'utilisateur qui, le plus souvent, est obligé d'accomplir plusieurs taches dans le même temps en fin de prélèvement ou de perfusion. Par exemple, l'utilisateur peut avoir à nettoyer à l'aide d'un coton le lieu de prélèvement ou de perfusion sur le corps du patient. L'utilisateur peut également avoir à tenir le coton comprimé sur le lieu de prélèvement ou de perfusion sur le corps du patient pour empêcher tout saignement à la suite d'un prélèvement ou d'une perfusion, et ce dès le retrait du dispositif de prélèvement ou de perfusion. La tenue de ce coton peut en effet ne pas être assurée par le patient lui-même dans le cas où celui-ci est inconscient par exemple.

Sur les figures 1, 3 et 4 du premier mode de réalisation de l'invention, le dispositif de prélèvement ou de perfusion à moyen de protection comporte des moyens de verrouillage du tube de protection 5 en position distale de protection et en position proximale de retrait.

Les moyens de verrouillage dans la position proximale de retrait comportent :
- une première encoche 5c annulaire périphérique pratiquée sur le tube de protection 5 au voisinage d'une extrémité distale 5b du tube de protection 5,
- des pattes de verrouillage 11 élastiques radiales s'étendant radialement depuis une extrémité distale 4b du corps externe 4, et venant se loger dans la première encoche 5c annulaire périphérique lorsque le tube de protection 5 est en position proximale de retrait (figure 1).

Les moyens de verrouillage dans la position distale de protection comportent :
- un épaulement 12 à face radiale 13 tournée vers l'extrémité proximale de préhension 5a du tube de protection, pratiqué sur le tube de protection 5 au voisinage de l'extrémité proximale de préhension 5a du tube de protection 5,
- des pattes de verrouillage 11 élastiques radiales s'étendant radialement depuis l'extrémité distale 4b du corps externe 4, et venant se loger sur l'épaulement 12 lorsque le tube de protection 5 est en position distale de protection (figures 3 et 4).

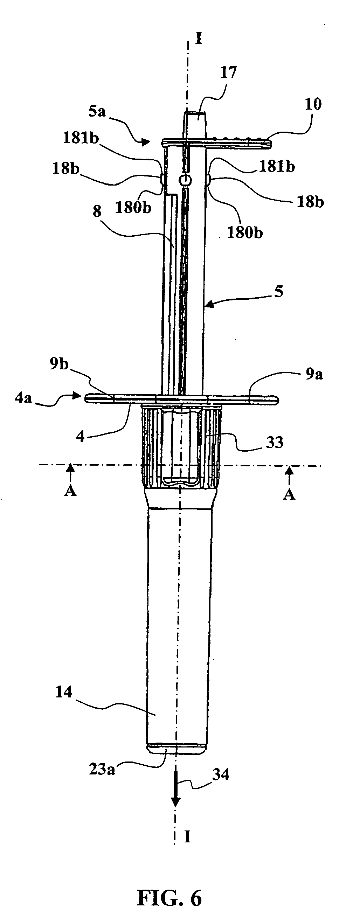

Sur les figures 6, 7, 8, 9 et 11 du second mode de réalisation de l'invention, les moyens de verrouillage du tube de protection 5 en position distale de protection et en position proximale de retrait sont d'un autre type.

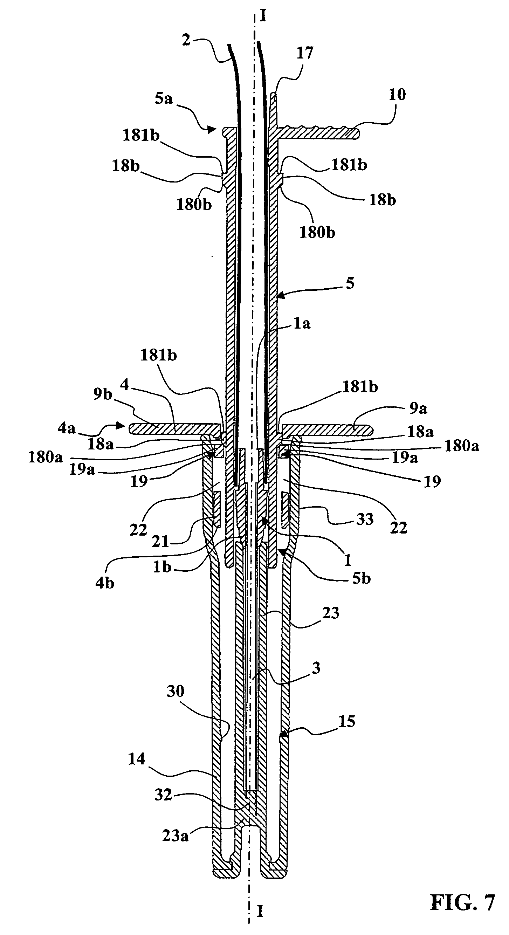

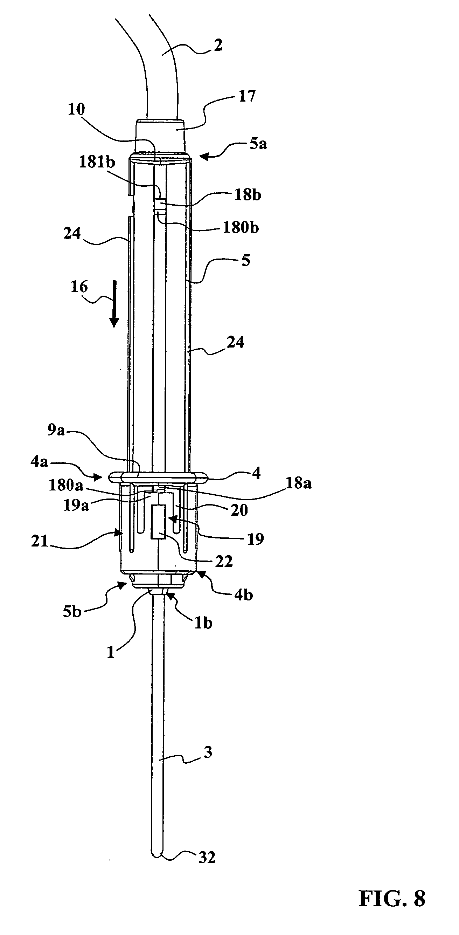

Ces moyens de verrouillage dans la position proximale de retrait comportent :
- deux premiers plots d'arrêt 18a s'étendant radialement à l'écart du tube de protection 5 au voisinage de l'extrémité distale 5b du tube de protection 5,
- deux pattes de verrouillage 19 longitudinales dans deux lumières 20 (figure 8) de la paroi périphérique tubulaire 21 du corps externe 4, aptes à se défléchir élastiquement radialement, à extrémité libre 19a orientée vers l'extrémité proximale 4a du corps externe 4, aptes à venir en butée contre le premier plot d'arrêt 18a lorsque le tube de protection 5 est en position proximale de retrait (figures 7 et 8).

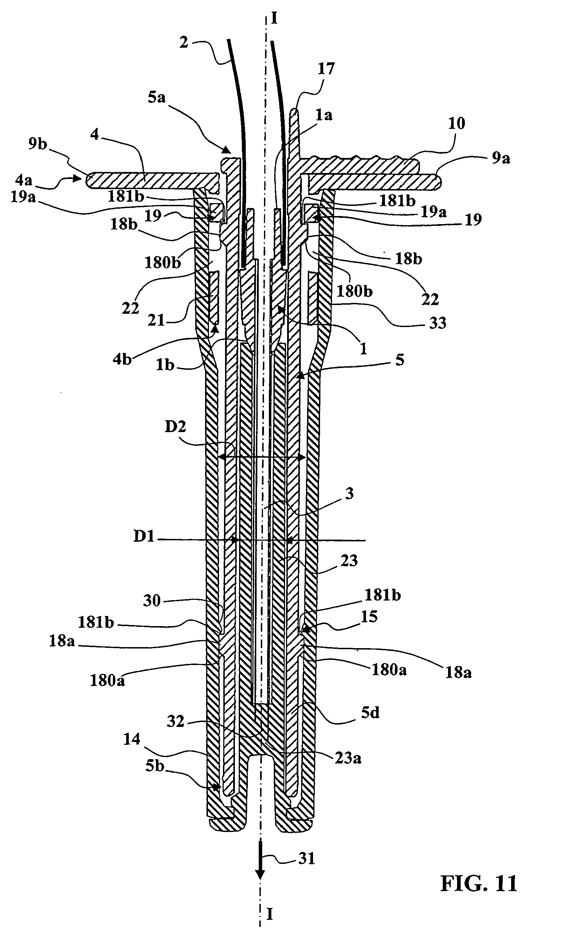

Ces moyens de verrouillage dans la position distale de protection comportent :
- deux seconds plots d'arrêt 18b s'étendant radialement à l'écart du tube de protection 5 au voisinage de l'extrémité proximale 5a du tube de protection 5,
- des lumières 22 dans les pattes de verrouillage 19, aptes à recevoir les seconds plots d'arrêt 18b lorsque le tube de protection 5 est en position distale de protection (figure 11).

De tels moyens de verrouillage en position proximale de retrait et en position distale de protection sont faciles à réaliser par injection plastique. Les pattes de verrouillage 19, les lumières 20 et 22 peuvent être réalisées à l'aide de tiroirs transversaux lors de l'injection plastique.

Les moyens de verrouillage dans la position proximale de retrait permettent d'empêcher tout coulissement intempestif du tube lorsque l'aiguille 3 est engagée dans le corps d'un patient. Le coulissement intempestif du tube de protection 5 pourrait en effet alors venir en conflit avec le corps du patient et être source de désagrément pour celui-ci lors du prélèvement ou de la perfusion.

Les moyens de verrouillage dans la position distale de protection permettent également d'éviter efficacement tout risque de coulissement intempestif du tube de protection 5 pouvant mener à découvrir l'aiguille 3 après usage une fois que le tube de protection 5 a été disposé en position distale de protection. On empêche ainsi tout contact ultérieur même accidentel avec l'aiguille 3 souillée par du sang du patient.

Il est particulièrement important, dans le milieu médical réalisant des prélèvements ou des perfusions, de s'assurer que le matériel ayant servi une première fois ne soit jamais réutilisé pour un autre patient, afin d'éviter tout risque de contamination entre les différents patients. Il est alors particulièrement utile de disposer d'un dispositif de prélèvement ou de perfusion à usage unique, et c'est pourquoi le dispositif de prélèvement ou de perfusion à moyen de protection selon l'invention peut avantageusement empêcher toute tentative de réutilisation ultérieure une fois que le tube de protection 5 a été disposé en position distale de protection suite à une première utilisation.

A cet effet, on observe sur les figures 1, 3 et 4 du premier mode de réalisation de l'invention que les pattes de verrouillage 11 élastiques radiales se développent de façon oblique par rapport à l'axe longitudinal I-I. Une telle disposition permet un coulissement unidirectionnel du tube de protection 5 depuis sa position proximale de retrait vers sa position distale de protection, et interdit tout coulissement du tube de protection 5 depuis sa position distale de protection vers sa position proximale de retrait.

On observe sur les figures 7 et 11 du second mode de réalisation de l'invention que les premiers et seconds plots d'arrêt 18a et 18b comportent des faces distales obliques 180a et 180b qui peuvent provoquer la déflection latérale élastique des pattes de verrouillage 19 lors du coulissement longitudinal d'axe |-| du tube de protection 5.

Les premiers et seconds plots d'arrêt 18a et 18b comportent en outre un épaulement proximal 181a et 181 b à face tournée vers l'extrémité proximale 5a du tube de protection 5. Les épaulements 181 a et 181b permettent de n'autoriser qu'un coulissement unidirectionnel du tube de protection 5 depuis sa position proximale de retrait vers sa position distale de protection.

L'épaulement 181b interdit tout coulissement du tube de protection 5 depuis sa position distale de protection (figure 11) vers sa position proximale de retrait (figure 7). Ceci garantit un usage unique du dispositif de prélèvement ou de perfusion selon l'invention.

Lorsque le tube de protection 5 est en position distale de protection, on élimine tout risque pour le personnel médical de se blesser avec l'aiguille 3 contaminée par le sang du patient, et tout risque de réutilisation.

Même si les gouttes de sang pouvant tomber de l'aiguille 3 souillée par du sang du patient peuvent être pour la plupart recueillies par le tube de protection 5, il se peut cependant que certaines gouttes échappent à ce tube de protection 5 en passant dans la fente longitudinale 8 ou par l'extrémité distale 5b débouchante du tube de protection 5. Il n'est pas non plus exclu qu'un enfant vienne se blesser avec un tel dispositif de prélèvement ou de perfusion selon l'invention même lorsque le tube de protection 5 est en position distale de protection, en engageant un doigt de petite taille dans l'extrémité distale 5b débouchante du tube de protection 5. Le doigt engagé peut alors venir au contact de l'aiguille 3 qui a été préalablement souillée par du sang d'un patient.

Pour assurer cette étanchéité et éviter tout risque qu'un enfant se blesse, le dispositif de prélèvement ou de perfusion à moyen de protection selon l'invention peut avantageusement comporter un capuchon de protection 14 tubulaire borgne amovible, conformé pour s'emmancher sur l'extrémité distale 4b du corps externe 4 en entourant la partie dépassante d'aiguille 3, et de diamètre suffisant pour recevoir le tronçon distal 5d dépassant du tube de protection 5 en position distale de protection (figures 4 et 11).

Dans le second mode de réalisation illustré sur la figure 7, le capuchon de protection 14 est emmanché sur le corps externe 4 alors que les extrémités libres 19a des pattes de verrouillage 19 sont en butée contre les faces distales obliques 180a des premiers plots d'arrêt 18a. Du fait de la présence du capuchon de protection 14, les pattes de verrouillage 19 ne peuvent se défléchir élastiquement radialement. On empêche ainsi tout coulissement axial du tube de protection 5 avant l'utilisation effective du dispositif de prélèvement ou de perfusion qui débute par le retrait du capuchon de protection 14 du corps externe 4.

Après utilisation et après avoir emmanché le capuchon de protection 14 sur l'extrémité distale 4b du corps externe 4 (figures 4 et 11), le capuchon de protection 14 tubulaire borgne s'oppose à l'expansion radiale élastique des pattes de verrouillage 11 ou 19, et contribue ainsi à interdire le coulissement du tube de protection depuis sa position distale de protection vers sa position proximale de retrait.

Pour toujours plus de sécurité, il est souhaitable que le capuchon de protection 14 ne puisse que difficilement, voire jamais se retirer une fois que celui-ci a été emmanché sur l'extrémité distale 4b du corps externe 4.

Pour ce faire, le capuchon de protection 14 tubulaire borgne comporte des moyens de retenue 15 du tube de protection 5 en position distale de protection.

Sur la figure 4, les moyens de retenue 15 du tube de protection 5 sont prévus dans le fond du capuchon et sont conformés pour venir s'engager dans la première encoche 5c annulaire périphérique du tube de protection 5 lorsque le tube de protection 5 est en position distale de protection et que le capuchon de protection 14 est emmanché sur l'extrémité distale 4b du corps externe 4.

Les moyens de retenue 15 du capuchon de protection 14 peuvent également être conformés d'une manière analogue aux pattes de verrouillage 11, pour assurer un déplacement unidirectionnel du capuchon de protection 14 par rapport au tube de protection 5 et pour empêcher tout mouvement de retrait du capuchon de protection 14 une fois que celui-ci a été emmanché sur l'extrémité distale 4b du corps externe 4. On garantit ainsi que le dispositif de prélèvement ou de perfusion ne pourra être utilisé qu'une et une seule fois pour un prélèvement ou une perfusion.

Sur la figure 11, les moyens de retenue 15 du tube de protection 5 comportent un bossage annulaire 30 interne au capuchon de protection 14. Le bossage annulaire 30 coopère avec les premiers plots d'arrêt 18a pour empêcher que le capuchon de protection 14, une fois emmanché sur l'extrémité distale 4b du corps externe 4, ne puisse être retiré dans les sens et direction définis par la flèche 31 lorsque le tube de protection 5 et en position distale de protection.

Lors de l'utilisation d'un dispositif de prélèvement ou de perfusion à moyen de protection selon l'invention, l'utilisateur (un infirmier par exemple) retire éventuellement le capuchon de protection 14, puis engage l'aiguille 3 dans le corps du patient, le tube de protection 5 étant et restant en position proximale de retrait (figures 1 et 8). La ligne de perfusion ou de prélèvement 2 est alors reliée à un tube de prélèvement ou à une poche de perfusion.

Une fois la perfusion ou le prélèvement effectué, l'utilisateur tient d'une main le dispositif de prélèvement ou de perfusion au niveau de l'extrémité proximale 4a du corps externe 4, avec un doigt en appui sous la patte de préhension radiale 9b et avec un autre doigt en appui sous la patte de préhension radiale 9a. L'utilisateur retire alors l'aiguille 3 du corps du patient qui est souillée par du sang du patient. Pour éviter tout risque de contamination ou de blessure par l'aiguille 3 souillée par le sang du patient, l'utilisateur applique alors immédiatement une poussée axiale sur la patte radiale de poussée 10 au moyen du pouce de la même main pour déplacer axialement le tube de protection 5 depuis sa position proximale de retrait (figures 1 et 8) jusqu'à sa position distale de protection (figures 3 et 11) selon un mouvement illustré par la flèche 16. L'utilisateur manipule ainsi le tube de protection 5 en agissant à l'écart de l'extrémité dangereuse de l'aiguille 3, et selon un mouvement qui ne va pas à l'encontre de l'extrémité dangereuse de l'aiguille 3. L'aiguille 3 est alors protégée et il n'y a plus aucun risque de blessure ou de contamination par cette aiguille 3 souillée par du sang du patient. L'utilisateur n'a eu à utiliser qu'une seule de ses mains pour à la fois retirer le dispositif de prélèvement ou de perfusion selon l'invention et pour protéger immédiatement l'aiguille 3 souillée par du sang du patient. Il lui a fallu pour cela effectuer un mouvement classique et bien connu pour lui, puisqu'utilisé pour la manipulation de seringues classiques, ce qui assure qu'il n'y ait aucun risque de mauvaise manipulation du dispositif de prélèvement ou de perfusion à moyen de protection selon l'invention.

Dans le second mode de réalisation de l'invention, il est prévu que l'extrémité proximale de préhension 5a du tube de protection 5 comporte une excroissance 17 prolongeant longitudinalement en partie le tube de protection 5 au-delà de la patte radiale de poussée 10. L'excroissance 17 s'étend depuis la partie au moins de la circonférence du tube de protection 5 depuis laquelle s'étend radialement la patte radiale de poussée 10. Lorsque l'utilisateur applique une poussée axiale sur la patte radiale de poussée 10 au moyen de son pouce, l'excroissance 17 empêche que le pouce de l'utilisateur ne vienne écraser ou plier la ligne de perfusion ou de prélèvement 2 (figure 11). On évite ainsi efficacement de gêner la circulation de fluide entre l'aiguille 3 et le tube de prélèvement ou la poche de perfusion connectée à la ligne de perfusion ou de prélèvement 2.

Pendant que l'utilisateur retire le dispositif de prélèvement ou de perfusion à moyen de protection et protège l'aiguille 3 souillée par du sang du patient au moyen du tube de protection 5, celui-ci est à même, avec sa seconde main, d'effectuer une autre tâche comme celle de tenir comprimé un morceau de coton contre le lieu de perfusion ou de prélèvement du corps du patient.

Une fois en position distale de protection, le tube de protection 5 est verrouillé par les pattes de verrouillage 11 (figure 3) ou 19 (figure 11) et ne peut plus coulisser depuis sa position distale de protection vers sa position proximale de retrait. Les dispositifs de prélèvement ou de perfusion ayant ainsi servi une fois peuvent être facilement reconnus visuellement par les utilisateurs, et même si ceux-ci tentaient de les réutiliser, les pattes de verrouillage 11 ou 19 empêchant le coulissement du tube de protection 5 rendraient la tentative impossible.

Enfin, en vue d'assurer une étanchéité parfaite et une sécurité complète du dispositif selon l'invention, l'utilisateur peut venir emmancher sur l'extrémité distale 4b du corps externe 4 le capuchon de protection 14. Celui-ci se verrouille par les moyens de retenue 15 dans la première encoche 5c annulaire périphérique du tube de protection 5 (figure 4) ou par la coopération du bossage annulaire 30 avec les premiers plots d'arrêt 18a (figure 11). Il est alors impossible de retirer le capuchon de protection 14 une fois que celui-ci a été emmanché une première fois sur l'extrémité distale 4b du corps externe 4.

Les conditions de sécurité et d'hygiène exigées dans le milieu médical sont donc parfaitement assurées par le dispositif de prélèvement ou de perfusion à moyen de protection selon l'invention, qui s'utilise de façon très simple à l'aide de gestes très usités dans le milieu médical. De la sorte, il n'y a aucun risque de blessure ou d'erreur de manipulation pour le personnel médical.

Une étanchéité parfaite est obtenue dans le second mode de réalisation. On voit plus particulièrement sur la figure 11 que le capuchon de protection 14 du second mode de réalisation de l'invention comporte un capuchon tubulaire 23, solidaire du fond du capuchon de protection 14, de diamètre extérieur D1 inférieur au diamètre intérieur D2 du tube de protection 5 et apte à venir recouvrir la partie dépassante d'aiguille 3 en venant en butée contre l'extrémité distale 1b du corps central 1.

On assure ainsi une étanchéité au voisinage de l'extrémité distale 1b du corps central 1 selon une portion annulaire du capuchon tubulaire 23 qui vient s'emmancher sur le corps central 1. On empêche ainsi tout liquide présent dans le capuchon tubulaire 23 de se répandre à l'extérieur et de contaminer l'environnement extérieur.

Dans le second mode de réalisation illustré sur la figure 11, le capuchon tubulaire 23 est en élastomère, et le biseau 32 de l'aiguille 3 vient se planter dans la paroi de fond 23a du capuchon tubulaire 23 sans la traverser lorsque le capuchon de protection 14 est emmanché sur l'extrémité distale 4b du corps externe 4. L'étanchéité du dispositif de prélèvement ou de perfusion est ainsi parfaite.

Comme l'illustre la figure 7, le biseau 32 de l'aiguille 3 est également planté dans la paroi de fond 23a du capuchon tubulaire 23 lorsque le tube de protection 5 est en position proximale de retrait et que le dispositif de prélèvement ou de perfusion est sur le point d'être utilisé. Le personnel hospitalier a alors déjà branché la ligne de perfusion 2 sur l'extrémité proximale 1a du corps central 1, et du liquide de la poche de perfusion remplit alors la ligne de perfusion 2 et l'aiguille 3. Le biseau 32 de l'aiguille 3 étant planté dans la paroi de fond 23a du capuchon tubulaire 23, le liquide présent dans l'aiguille 3 et dans la poche de perfusion ne peut s'écouler et contaminer l'environnement extérieur. On facilite ainsi grandement le travail du personnel hospitalier en lui permettant de connecter une poche de perfusion via une ligne de perfusion 2 sans risque de perte de liquide de perfusion, et sans risque de contamination de l'environnement.

Avantageusement, le corps central 1, le corps externe 4 et le capuchon de protection 14 peuvent être en matière plastique. L'emploi d'une telle matière permet d'alléger au maximum le dispositif de prélèvement ou de perfusion selon l'invention, de réaliser celui-ci de manière industrielle, rapide et économique. En outre, son poids faible contribuera à une bonne maniabilité.

Dans le second mode de réalisation de l'invention, on voit plus particulièrement sur la figure 6 que le tube de protection 5 comporte des reliefs de préhension 33 sur sa surface extérieure. Ces reliefs de préhension 33 facilitent la prise en main du capuchon de protection 14 par l'utilisateur en vue de retirer le capuchon de protection 14 dans les sens et direction illustrés par la flèche 34.

La présente invention n'est pas limitée aux modes de réalisation qui ont été explicitement décrits, mais elle en inclut les diverses variantes et généralisations contenues dans le domaine des revendications ci-après.

## Revendications

1. Dispositif de prélèvement de fluide corporel ou de perfusion à moyen de protection, comportant :
- un corps central (1) longitudinal creux, permettant le passage d'un fluide entre une première et une seconde extrémité, muni à son extrémité proximale (1a) de moyens de connexion à une ligne de perfusion ou de prélèvement, et solidaire d'une aiguille (3) coaxiale qui dépasse de son extrémité distale (1 b),
- des moyens pour recouvrir sélectivement la partie dépassante d'aiguille (3) après usage,
- un corps externe (4) longitudinal creux à paroi périphérique tubulaire (21), solidaire du corps central (1) et disposé de façon concentrique autour du corps central (1), et conformé pour constituer un moyen de préhension pour un utilisateur,
- un passage annulaire (6) prévu entre le corps central (1) et le corps externe (4),
- un tube de protection (5) ouvert à son extrémité distale (5b) et monté à coulissement longitudinal dans le passage annulaire (6) entre le corps externe (4) et le corps central (1), muni d'une extrémité proximale de préhension (5a) pour être déplacé sélectivement entre d'une part une position proximale de retrait dans laquelle l'aiguille (3) n'est pas ou très peu recouverte par le tube de protection de telle sorte que celle-ci est utilisable pour un prélèvement ou une perfusion, et d'autre part une position distale de protection dans laquelle le tube de protection (5) recouvre l'aiguille (3) sur toute sa longueur,
- au moins un pont radial (7) de fixation, rendant le corps externe (4) solidaire du corps central (1) en se développant radialement depuis le corps central (1) jusqu'au corps externe (4),
- au moins une fente longitudinale (8), sur une partie au moins de la longueur du tube de protection (5), dans laquelle coulisse ledit au moins un pont radial (7) de fixation lors du mouvement de coulissement du tube de protection (5) par rapport aux corps central (1) et externe (4),
**caractérisé en ce que :**
- le corps central (1), le pont radial (7) de fixation et le corps externe (4) sont d'une seule pièce,
- la fente longitudinale (8) se développe jusqu'à l'extrémité distale (5b) du tube de protection (5).

2. Dispositif selon la revendication 1, **caractérisé en ce que** le corps externe (4) comporte au moins une patte de préhension radiale (9a, 9b) s'étendant radialement à l'écart du corps externe (4) et prévue au voisinage de l'extrémité proximale (4a) du corps externe (4).

3. Dispositif selon l'une des revendications 1 ou 2, **caractérisé en ce que** l'extrémité proximale de préhension (5a) du tube de protection (5) comporte au moins une patte radiale de poussée (10) s'étendant radialement à l'écart du tube de protection (5) depuis une partie au moins de la circonférence du tube de protection (5).

4. Dispositif selon la revendication 3, **caractérisé en ce que :**
- l'extrémité proximale de préhension (5a) du tube de protection (5) comporte une excroissance (17) prolongeant longitudinalement au moins en partie le tube de protection (5) au-delà de la patte radiale de poussée (10),
- l'excroissance (17) s'étend depuis la partie au moins de la circonférence du tube de protection (5) depuis laquelle s'étend radialement la patte radiale de poussée (10).

5. Dispositif selon l'une quelconque des revendications 1 à 4, **caractérisé en ce qu'**il comporte des moyens de verrouillage du tube de protection (5) en position distale de protection et/ou en position proximale de retrait.

6. Dispositif selon la revendication 5, **caractérisé en ce que** les moyens de verrouillage dans la position proximale de retrait comportent :
- une première encoche (5c) annulaire périphérique pratiquée sur le tube de protection (5) au voisinage d'une extrémité distale (5b) du tube de protection (5),
- au moins une patte de verrouillage (11) élastique radiale s'étendant radialement depuis une extrémité distale (4b) du corps externe (4), et venant s'engager dans la première encoche (5c) annulaire périphérique lorsque le tube de protection (5) est en position proximale de retrait.

7. Dispositif selon la revendication 5, **caractérisé en ce que** les moyens de verrouillage dans la position distale de protection comportent :
- une seconde encoche annulaire périphérique ou un épaulement (12) à face (13) tournée vers l'extrémité proximale de préhension (5a), pratiquée sur le tube de protection (5) au voisinage de l'extrémité proximale de préhension (5a) du tube de protection (5),
- au moins une patte de verrouillage (11) élastique radiale s'étendant radialement depuis l'extrémité distale (4b) du corps externe (4), et venant se loger dans la seconde encoche annulaire périphérique ou sur l'épaulement (12) lorsque le tube de protection (5) est en position distale de protection.

8. Dispositif selon la revendication 5, **caractérisé en ce que** les moyens de verrouillage dans la position proximale de retrait comportent :
- au moins un premier plot d'arrêt (18a) s'étendant radialement à l'écart du tube de protection (5) au voisinage de l'extrémité distale (5b) du tube de protection (5),
- au moins une patte de verrouillage (19) longitudinale dans une lumière (20) de la paroi périphérique tubulaire (21) du corps externe (4), apte à se défléchir élastiquement radialement, à extrémité libre (19a) orientée vers l'extrémité proximale (4a) du corps externe (4) apte à venir en butée contre le premier plot d'arrêt (18a) lorsque le tube de protection (5) est en position proximale de retrait.

9. Dispositif selon la revendication 8, **caractérisé en ce que** les moyens de verrouillage dans la position distale de protection comportent :
- au moins un second plot d'arrêt (18b) s'étendant radialement à l'écart du tube de protection (5) au voisinage de l'extrémité proximale (5a) du tube de protection (5),
- une lumière (22) dans la patte de verrouillage (19), apte à recevoir le second plot d'arrêt (18b) lorsque le tube de protection (5) est en position distale de protection.

10. Dispositif selon l'une quelconque des revendications 1 à 9, **caractérisé en ce qu'**il comporte un capuchon de protection (14) tubulaire borgne amovible conformé pour s'emmancher sur l'extrémité distale (4b) du corps externe (4) en entourant la partie dépassante d'aiguille (3), et de diamètre suffisant pour recevoir le tronçon distal (5d) dépassant du tube de protection (5) en position distale de protection.

11. Dispositif selon la revendication 10, **caractérisé en ce que,** emmanché sur l'extrémité distale du corps externe, le capuchon de protection (14) tubulaire borgne s'oppose à l'expansion radiale élastique de ladite au moins une patte de verrouillage (11, 19), et interdit ainsi le coulissement du tube de protection (5).

12. Dispositif selon l'une des revendications 10 ou 11, **caractérisé en ce que** le capuchon de protection (14) tubulaire borgne comporte dans son fond des moyens de retenue (15) du tube de protection (5) en position distale de protection.

13. Dispositif selon la revendication 12, **caractérisé en ce que** les moyens de retenue (15) du tube de protection (5) sont conformés pour venir s'engager dans une première encoche (5c) annulaire périphérique du tube de protection (5) lorsque le tube de protection (5) est en position distale de protection et que le capuchon de protection (14) est emmanché sur l'extrémité distale (4b) du corps externe (4).

14. Dispositif selon l'une quelconque des revendications 10 à 13, **caractérisé en ce que** le capuchon de protection (14) comporte des reliefs de préhension (33) sur sa surface extérieure.

15. Dispositif selon l'une quelconque des revendications 1 à 14, **caractérisé en ce que** le corps central (1), le corps externe (4) et le capuchon de protection (14) sont en matière plastique.

16. Dispositif selon l'une quelconque des revendications 10 à 15, **caractérisé en ce que** le capuchon de protection (14) comporte un capuchon tubulaire (23), solidaire du fond du capuchon de protection (14), de diamètre extérieur (D1) inférieur au diamètre intérieur (D2) du tube de protection (5) et apte à venir recouvrir la partie dépassante d'aiguille (3) en venant en butée contre l'extrémité distale (1b) du corps central (1).

17. Dispositif selon la revendication 16, **caractérisé en ce que** le capuchon tubulaire (23) est en élastomère et **en ce que** l'aiguille (3) vient se planter dans sa paroi de fond (23a) sans la traverser lorsque le capuchon de protection (14) est emmanché sur l'extrémité distale (4b) du corps externe (4).

18. Dispositif selon l'une quelconque des revendications 1 à 15, **caractérisé en ce que** le tube de protection (5) comporte au moins une nervure longitudinale (24) apte à coopérer avec au moins une rainure longitudinale (25) du corps externe (4) pour assurer le guidage du tube de protection (5) lors de son coulissement dans le corps externe (4).

## Claims

1. Bodily fluid sampling or transfusion device incorporating protection means, said device including:
- a longitudinal hollow central body (1) that enables a fluid to flow between a first end and a second end, is provided at its proximal end (1a) with means for connecting it to a sampling or transfusion line and is attached to a coaxial needle (3) that projects from its distal end (1b),
- means for selectively covering the projecting portion of the needle (3) after use,
- a longitudinal hollow external body (4) that has a peripheral tubular wall (21), is attached to the central body (1), is disposed concentrically around the central body (1) and is conformed to constitute holding means for a user to hold onto,
- an annular passage (6) between the central body (1) and the external body (4),
- a protective tube (5), that is open at its distal end (5b) and that can slide longitudinally in the annular passage (6) between the external body (4) and the central body (1) and has a proximal holding end (5a) for moving it selectively between, on one hand a proximal retracted position in which the needle (3) is not covered by the protective tube or only very slightly covered thereby so that it can be used to draw a sample or to administer a transfusion, and on the other hand a distal protection position in which the protective tube (5) covers the needle (3) over its entire length,
- at least one radial fixing bridge (7) attaching the external body (4) to the central body (1) and extending radially from the central body (1) to the external body (4),
- at least one longitudinal slot (8) that extends over at least a portion of the length of the protective tube (5) and in which said at least one radial fixing bridge (7) slides during sliding movement of the protective tube (5) relative to the central body (1) and the external body (4), **characterized in that:**
- the central body (1), the radial fixing bridge (7) and the external body (4) are in one piece,
- the longitudinal slot (8) extends as far as the distal end (5b) of the protective tube (5).

2. Device according to claim 1, **characterized in that** the external body (4) includes at least one radial holding tab (9a, 9b) extending radially away from the external body (4) in the vicinity of the proximal end (4a) of the external body (4).

3. Device according to either claim 1 or claim 2, **characterized in that** the proximal holding end (5a) of the protective tube (5) includes at least one radial pusher tab (10) extending radially away from the protective tube (5) from at least a portion of the circumference of the protective tube (5).

4. Device according to claim 3, **characterized in that:**
- the proximal holding end (5a) of the protective tube (5) includes a protuberance (17) extending longitudinally along at least a portion of the protective tube (5) beyond the radial pusher tab (10),
- the protuberance (17) extends from said at least a portion of the circumference of the protective tube (5) from which the radial pusher tab (10) extends radially.

5. Device according to any one of claims 1 to 4, **characterized in that** it includes means for locking the protective tube (5) in the distal protection position and/or in the proximal retracted position.

6. Device according to claim 5, **characterized in that** the proximal retracted position locking means include:
- a first annular peripheral notch (5c) on the protective tube (5) in the vicinity of the distal end (5b) of the protective tube (5),
- at least one elastic radial locking lug (11) extending radially from a distal end (4b) of the external body (4) and engaging in the first annular peripheral notch (5c) when the protective tube (5) is in the proximal retracted position.

7. Device according to claim 5, **characterized in that** the distal protection position locking means include:
- a second annular peripheral notch or a shoulder (12) with a face (13) facing toward the proximal holding end (5a) on the protective tube (5) in the vicinity of the proximal holding end (5a) of the protective tube (5),
- at least one elastic radial locking lug (11) extending radially from the distal end (4b) of the external body (4) and accommodated in the second annular peripheral notch or located over the shoulder (12) when the protective tube (5) is in the distal protection position.

8. Device according to claim 5, **characterized in that** the proximal retracted position locking means include:
- at least one first stop (18a) extending radially away from the protective tube (5) in the vicinity of the distal end (5b) of the protective tube (5),
- at least one longitudinal locking lug (19) in an opening (20) in the peripheral tubular wall (21) of the external body (4) that is adapted to be flexed elastically in the radial direction and has a free end (19a) oriented toward the proximal end (4a) of the external body (4) adapted to abut against the first stop (18a) when the protective tube (5) is in the proximal retracted position.

9. Device according to claim 8, **characterized in that** the distal protection position locking means include:
- at least one second stop (18b) extending radially away from the protective tube (5) in the vicinity of the proximal end (5a) of the protective tube (5),
- an opening (22) in the locking lug (19) adapted to receive the second stop (18b) when the protective tube (5) is in the distal protection position.

10. Device according to any one of claims 1 to 9, **characterized in that** it includes a removable blind tubular protective cap (14) that is conformed to fit over the distal end (4b) of the external body (4) around the projecting portion of the needle (3) and is of sufficient diameter to receive the projecting distal portion (5d) of the protective tube (5) in the distal protection position.

11. Device according to claim 10, **characterized in that,** when fitted over the distal end of the external body, the blind tubular protective cap (14) opposes elastic radial expansion of said at least one locking lug (11, 19) and thereby prevents sliding of the protective tube (5).

12. Device according to either claim 10 or claim 11, **characterized in that** the blind tubular protective cap (14) includes at its closed end retaining means (15) for retaining the protective tube (5) in the distal protection position.

13. Device according to claim 12, **characterized in that** the retaining means (15) for retaining the protective tube (5) are conformed to engage in a first annular peripheral notch (5c) of the protective tube (5) when the protective tube (5) is in the distal protection position and the protective cap (14) is fitted over the distal end (4b) of the external body (4).

14. Device according to any one of claims 10 to 13, **characterized in that** the protective cap (14) has raised patterns (33) on its exterior surface for holding it by.

15. Device according to any one of claims 1 to 14, **characterized in that** the central body (1), the external body (4) and the protective cap (14) are of plastic material.

16. Device according to any one of claims 10 to 15, **characterized in that** the protective cap (14) includes a tubular cap (23) that is attached to the closed end of the protective cap (14), has an exterior diameter (D1) less than the interior diameter (D2) of the protective tube (5) and is adapted to cover the projecting portion of the needle (3) upon abutting against the distal end (1b) of the central body (1).

17. Device according to claim 16, **characterized in that** the tubular cap (23) is of elastomer and **in that** the needle (3) is embedded in its closed end wall (23a) without passing through it when the protective cap (14) is fitted over the distal end (4b) of the external body (4).

18. Device according to any one of claims 1 to 15, **characterized in that** the protective tube (5) includes at least one longitudinal rib (24) adapted to cooperate with at least one longitudinal groove (25) on the external body (4) to guide the protective tube (5) when it slides in the external body (4).

## Patentansprüche

1. Vorrichtung zur Entnahme von Körperflüssigkeit oder zur Perfusion mit einer Schutzeinrichtung, umfassend:
- einen longitudinalen, zentralen Hohlkörper (1) für den Durchlauf einer Flüssigkeit zwischen einem ersten und einem zweiten Ende, der an seinem proximalen Ende (1a) Mittel zum Anschluss an eine Perfusions- oder Entnahmeleitung hat und fest mit einer koaxialen Nadel (3) verbunden ist, die von seinem distalen Ende (1b) vorsteht,
- Mittel zur selektiven Abdeckung des vorstehenden Abschnitts der Nadel (3) nach dem Gebrauch,
- einen longitudinalen, hohlen Außenkörper (4) mit einer rohrförmigen Umfangswand (21), der fest mit dem zentralen Körper (1) verbunden ist, diesen konzentrisch umschließt und als ein Mittel zum Erfassen durch den Benutzer ausgebildet ist,
- einen Ringspalt (6) zwischen dem zentralen Körper (1) und dem Außenkörper (4),
- ein Schutzrohr (5), das an seinem distalen Ende (5b) offen und in Längsrichtung innerhalb des Ringspaltes (6) zwischen dem Außenkörper (4) und dem zentralen Körper (1) verschiebbar ist und an dem ein proximales Griffende (5a) ausgebildet ist zum selektiven Verschieben zwischen einerseits einer proximalen Rückzugsstellung, in der die Nadel (3) nicht oder nur wenig von dem Schutzrohr abgedeckt ist, so dass sie für die Entnahme oder die Perfusion einsetzbar ist, und andererseits einer distalen Schutzstellung, in der das Schutzrohr (5) die Nadel (3) über ihre gesamte Länge abdeckt,
- wenigstens eine radiale Fixierbrücke (7), die den Außenkörper (4) mit dem zentralen Körper (1) verbindet sich dabei von dem zentralen Körper (1) radial bis zu dem Außenkörper (4) erstreckt,
- wenigstens einen Längsschlitz (8) auf wenigstens einem Abschnitt der Länge des Schutzrohres (5), in dem die wenigstens eine radiale Fixierbrücke (7) bei der Verschiebebewegung des Schutzrohres (5) relativ zum zentralen Körper (1) und zum Außenkörper (4) gleitet,
**dadurch gekennzeichnet, dass:**
- der zentrale Körper (1), die radiale Fixierbrücke (7) und der Außenkörper (4) aus einem einzigen Teil bestehen,
- der Längsschlitz (8) sich bis zum distalen Ende (5b) des Schutzrohres (5) erstreckt.

2. Vorrichtung nach Anspruch 1, **dadurch gekennzeichnet, dass** der Außenkörper (4) wenigstens eine radiale Grifflasche (9a, 9b) hat, die sich radial von dem Außenkörper (4) erstreckt und in der Nähe des proximalen Endes (4a) des Außenkörpers (4) vorgesehen ist.

3. Vorrichtung nach einem der Ansprüche 1 oder 2, **dadurch gekennzeichnet, dass** das proximale Griffende (5a) des Schutzrohres (5) wenigstens eine radiale Drucklasche (10) hat, die von wenigstens einem Teil des Umfangs des Schutzrohres (5) radial von diesem absteht.

4. Vorrichtung nach Anspruch 3, **dadurch gekennzeichnet, dass:**
- das proximale Griffende (5a) des Schutzrohres (5) einen Vorsprung (17) aufweist, der das Schutzrohr (5) wenigstens teilweise longitudinal über die radiale Drucklasche (10) hinaus verlängert,
- und sich der Vorsprung (17) von wenigstens dem Teil des Umfangs des Schutzrohres (5) erstreckt, von dem die radiale Drucklasche (10) radial absteht.

5. Vorrichtung nach einem der Ansprüche 1 bis 4, **dadurch gekennzeichnet, dass** die Mittel zur Verriegelung des Schutzrohres (5) in der distalen Schutzstellung und/oder in der proximalen Rückzugsstellung aufweist.

6. Vorrichtung nach Anspruch 5, **dadurch gekennzeichnet, dass** die Mittel zur Verriegelung in der proximalen Rückzugsstellung umfassen:
- eine erste umlaufende Ringnut (5c), die in der Nähe des distalen Endes (5b) des Schutzrohres (5) in dieses eingearbeitet ist,
- wenigstens eine radiale, elastische Rastklinke (11), die sich von einem distalen Ende (4b) des Außenkörpers (4) in radialer Richtung erstreckt und in die erste Ringnut (5c) eingreift, wenn sich das Schutzrohr (5) in der proximalen Rückzugsstellung befindet.

7. Vorrichtung nach Anspruch 5, **dadurch gekennzeichnet, dass** die Verriegelungsmittel in der distalen Schutzstellung umfassen:
- eine zweite umlaufende Ringnut oder Schulter (12) mit einer zum proximalen Griffende (5a) gerichteten Fläche (13), die in das Schutzrohr (5) in Nähe seines proximalen Griffendes (5a) eingearbeitet ist,
- wenigstens eine radiale, elastische Rastklinke (11), die sich von dem distalen Ende (4b) des Außenkörpers (4) erstreckt und in die zweite umlaufende Ringnut eingreift oder an der Schulter (12) zur Anlage kommt, wenn sich das Schutzrohr (5) in der distalen Schutzstellung befindet.

8. Vorrichtung nach Anspruch 5, **dadurch gekennzeichnet, dass** die Verriegelungsmittel in der proximalen Rückzugsstellung umfassen:
- wenigstens einen ersten Haltevorsprung (18a), der sich in der Nähe des distalen Endes (5b) des Schutzrohres (5) von diesem in radialer Richtung erstreckt,
- wenigstens eine longitudinale Rastklinke (19) in einer Öffnung (20) der rohrförmigen Umfangswand (21) des Außenkörpers (4), die radial elastisch nachgiebig ist und ein freies Ende (19a) hat, das zum proximalen Ende (4a) des Außenkörpers (4) weist und zum Anschlag an dem ersten Haltevorsprung (18a) kommt, wenn sich das Schutzrohr (5) in der proximalen Rückzugsstellung befindet.

9. Vorrichtung nach Anspruch 8, **dadurch gekennzeichnet, dass** die Verriegelungsmittel in der distalen Schutzstellung umfassen:
- wenigstens einen zweiten Haltevorsprung (18b), der sich in der Nähe des proximalen Endes (5a) des Schutzrohres (5) radial von diesem erstreckt,
- eine in die Rastklinke (19) eingearbeitete Öffnung (22), in die der zweite Haltevorsprung (18b) eingreift, wenn sich das Schutzrohr (5) in der distalen Schutzstellung befindet.

10. Vorrichtung nach einem der Ansprüche 1 bis 9, **dadurch gekennzeichnet, dass** sie eine abnehmbare, rohrförmige, einseitig geschlossene Schutzkappe (14) aufweist, die auf das distale Ende (4b) des Außenkörpers (4) aufsetzbar ist und dabei den überstehenden Abschnitt der Nadel (3) einschließt, wobei ihr Durchmesser so bemessen ist, dass er den distalen Endabschnitt (5d) aufnehmen kann, der von dem Schutzrohr (5) in seiner distalen Schutzstellung übersteht.

11. Vorrichtung nach Anspruch 10, **dadurch gekennzeichnet, dass** die rohrförmige, einseitig geschlossene Schutzkappe (14) in ihrer auf das distale Ende des Außenkörpers aufgesetzten Position einer elastischen, radialen Aufweitung der wenigstens einen Rastklinke (11, 19) entgegenwirkt und dadurch eine Verschiebung des Schutzrohres (5) verhindert.

12. Vorrichtung nach einem der Ansprüche 10 oder 11, **dadurch gekennzeichnet, dass** die rohrförmige einseitig geschlossene Schutzkappe (14) an ihrem Boden Mittel (15) zum Halten des Schutzrohres (5) in seiner distalen Schutzstellung aufweist.

13. Vorrichtung nach Anspruch 12, **dadurch gekennzeichnet, dass** die Haltemittel (15) für das Schutzrohr (5) so ausgebildet sind, dass sie in eine erste, umlaufende Ringnut (5c) des Schutzrohres (5) eingreifen, wenn sich dieses in seiner distalen Schutzstellung befindet, und dass die Schutzkappe (14) auf das distale Ende (4b) des Außenkörpers (4) aufgesetzt ist.

14. Vorrichtung nach einem der Ansprüche 10 bis 13, **dadurch gekennzeichnet, dass** die Schutzkappe (14) an ihrer Außenseite eine Griffrändelung (33) aufweist.

15. Vorrichtung nach einem der Ansprüche 1 bis 14, **dadurch gekennzeichnet, dass** der zentrale Körper (1), der Außenkörper (4) und die Schutzkappe (14) aus Kunststoff bestehen.

16. Vorrichtung nach einem der Ansprüche 10 bis 15, **dadurch gekennzeichnet, dass** die Schutzkappe (14) eine rohrförmige Kappe (23) hat, die einstückig mit dem Boden der Schutzkappe (14) ausgebildet ist und einen Außendurchmesser (D1) hat, der kleiner als der Innendurchmesser (D2) des Schutzrohres (5) ist, und die den überstehenden Abschnitt der Nadel (3) übergreifen kann und dabei zum Anschlag an dem distalen Ende (1b) des zentralen Körpers (1) kommt.

17. Vorrichtung nach Anspruch 16, **dadurch gekennzeichnet, dass** die rohrförmige Kappe (23) aus einem Elastomer besteht und dass die Nadel (3) in die Bodenwand (23a) der Kappe eindringt, ohne sie dabei zu durchstechen, wenn die Schutzkappe (14) auf das distale Ende (4b) des Außenkörpers (4) aufgesetzt ist.

18. Vorrichtung nach einem der Ansprüche 1 bis 15, **dadurch gekennzeichnet, dass** das Schutzrohr (5) wenigstens eine Längsrippe (24) für den Eingriff in wenigstens eine Längsnut (25) des Außenkörpers (4) aufweist, um die Führung des Schutzrohres (5) bei seiner Verschiebung in dem Außenkörper (4) zu gewährleisten.
